# EUROPEAN PATENT APPLICATION

(11) **EP 3 650 531 A1**
(43) Date of publication of application: **13.05.2020**
(21) Application number: 18205419.7
(22) Date of filing: 09.11.2018
(51) Int. Cl.: C12M 1/33, B01D 35/10, A61K 35/35, C12M 1/00, C12N 5/077

(54) **ADIPOSE TISSUE PROCESSING DEVICE**

(71) Applicant: DSM IP Assets B.V., 6411 TE Heerlen (NL)
(72) Inventor: FISHER, William T., 6100 AA ECHT (NL); DEKA, Amit, 6100 AA ECHT (NL)
(74) Representative: DSM Intellectual Property

(57) **Abstract**

In an embodiment, a device for processing adipose tissue comprises:
a. a rotatable chamber having a longitudinal axis around which the rotatable chamber is arranged to be rotated, and a first end and a second end, with a side wall extending therebetween, wherein said first end has a diameter smaller than a diameter of said second end, the rotatable chamber comprising within:
i. a fiber collector;
ii. a first outlet at a first radial distance from said longitudinal axis;
iii. a screen projecting from the second end and configured to restrict the passage of a constituent of adipose tissue therethrough, wherein the screen meets the second end at a second radial distance from the longitudinal axis, and wherein the second radial distance is less than the first radial distance;

b. a drive unit configured to rotate the rotatable chamber about the longitudinal axis, thereby producing a centrifugal field, whereupon when a sample of adipose tissue is present in the rotatable chamber, the sample stratifies into at least two constituent layers as a function of the differing specific gravities of the constituents.

## Description

### Field of the Invention

The present invention pertains to devices and methods for processing fat. Such devices may be useful in the field of, for example, plastic surgery.

### Background

Fat is removed and reinjected into the body in numerous plastic surgery methods. It is often desirable to remove fibers and liquids, which may contain free oil, tumescent fluid, and cell debris, from the fat. Removal of fibers facilitates reinjection of the fat by minimizing syringe and needle clogging.

One method of doing this is gravity decantation where the fat tissue and fluids, such as blood cells, separate over time. This method takes too much time and generally does not wash the fat. If one were to wash the fat, a second decantation step would likely be needed, adding further time to the procedure.

A second method is centrifuging the fat. This may remove liquid from the fat but does not generally remove fibers. Additionally, a separate washing step is required.

A third method is a device called the Revolve™ system by LifeCell Corporation. The Revolve™ system processes separates liquids, washes, and removes fibers in a single device. The device involves uses a vacuum source in combination with a manually operated tissue collector. The tissue collector comprises one or more toothed members configured to collect and retain fibers. Such devices are disclosed in WO2012/006587, WO2013/106655, WO2014/039697, WO2014/110448, and WO2015/035221.

Further examples of potentially relevant devices are disclosed in WO2012/067658, WO2013/123216, WO2014/011213, WO2014/0164815, WO2014/154990, US2015093362, WO2015117007, and WO2018/044791.

### Summary

Despite the above documents, there is a need in the art for improved processes and devices for fat processing.

In an embodiment, a device for processing adipose tissue comprises:
a. a rotatable chamber having a longitudinal axis around which the rotatable chamber is arranged to be rotated, and a first end and a second end, with a side wall extending therebetween, wherein said first end has a diameter smaller than a diameter of said second end, the rotatable chamber comprising within:
   i. a fiber collector;
   ii. a first outlet at a first radial distance from said longitudinal axis;
   iii. a screen projecting from the second end and configured to restrict the passage of a constituent of adipose tissue therethrough, wherein the screen meets the second end at a second radial distance from the longitudinal axis, and wherein the second radial distance is less than the first radial distance;
b. a drive unit configured to rotate the rotatable chamber about the longitudinal axis, thereby producing a centrifugal field, whereupon when a sample of adipose tissue is present in the rotatable chamber, the sample stratifies into at least two constituent layers as a function of the differing specific gravities of the constituents.

In an embodiment, a process for processing a sample comprises
a. introducing a sample into the rotatable chamber,
b. rotating the rotatable chamber, thereby stratifying the sample into constituents based on the density of the constituents,
c. removing a first fluid constituent from the sample via the first outlet in the rotatable chamber, and
d. recovering a desired constituent of the sample from the rotatable chamber.

The disclosed inventions may be beneficial over the prior art in that they may be more sterile, more self-contained, faster, more compact, easier to use, better at removing fibers, better at removing contaminants, better at removing oils or other liquids, or otherwise more efficacious than the prior art devices.

### Brief Description of the Drawings

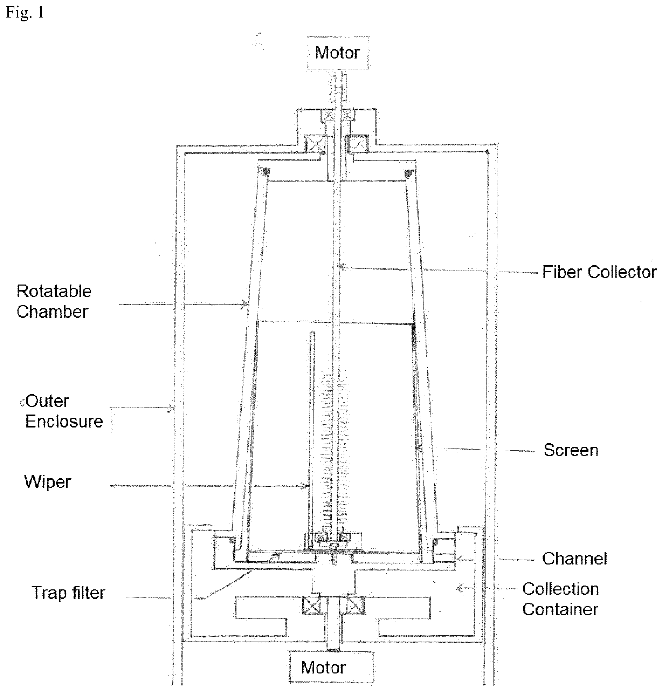

Fig. 1 is a cross-section of an embodiment of the invention.

### Description

The device comprises a processing unit. The processing unit comprises a rotatable chamber and optionally an outer enclosure. The rotatable chamber is arranged to contain a biologic mixture and has a central longitudinal axis about which the rotatable chamber is arranged to be rotated. The rotatable chamber may be contained within an outer enclosure to ensure that the operator cannot inadvertently touch the rotatable chamber. The outer enclosure may be arranged to receive a biologic mixture from the rotatable chamber and may be arranged coaxially upon the central longitudinal axis of the rotatable chamber.

The rotatable chamber comprises a sidewall and a base. The sidewall has a tapered inner surface. The tapered inner surface urges the liquid to move toward an outlet of the rotatable chamber. In an embodiment, the rotatable chamber has a first end and a second end, with a side wall extending therebetween, wherein said first end has a diameter smaller than a diameter of said second end. In an embodiment, the second end is the base.

The processing unit comprises a port to allow a sample, e.g. lipoaspirate, to be placed into the rotatable chamber. In an embodiment, the port is configured to receive the tip of a syringe or catheter. In an embodiment, the port comprises a cap that can be opened to allow input of the sample and closed to seal the processing unit.

The rotatable chamber comprises a fiber collector located within the rotatable chamber. The fiber collector is configured to collect fibers from the sample, such as collagen fibers in lipoaspirate. Such fibers may agglomerate and be wound on the fiber collector, such as the appearance of a string wound around the rotating brush of a vacuum cleaner.

In an embodiment, the fiber collector is rotatable. In an embodiment the fiber collector is rotatable about its central axis. In an embodiment the fiber collector is rotatable about the longitudinal axis of the rotatable chamber. In an embodiment, the fiber collector is connected to the same rotation source as the rotatable chamber. In an embodiment, the fiber collector is connected to the same rotation source as the rotatable chamber and is configured to rotate slower than the rotation source of the rotatable chamber. Such a configuration can be accomplished by e.g. gears.

In an embodiment, the fiber collector is connected to its own rotation source, as depicted in Fig. 1. In an embodiment, the rotation source coupled to the fiber collector is present at the top of the processing unit and may be decoupled from the fiber collector and reused.

In an embodiment, the fiber collector has the same revolution frequency as the rotatable chamber. In an embodiment, the fiber collector is configured to rotate and at a slower revolution frequency than the rotatable chamber. In an embodiment, the fiber collector is configured to rotate at a faster revolution frequency than the rotatable chamber. In an embodiment, the fiber collector is present coaxially with or proximate to the longitudinal axis of the rotatable chamber. In an embodiment, the fiber collectors outer edge is present at a fourth radial distance from the longitudinal axis. In an embodiment, the fiber collector is present at a fifth radial distance from the longitudinal axis.

In an embodiment, the fiber collector is a brush. In an embodiment, the brush comprises nylon bristles. In an embodiment, the fiber collector is a comb. The height of the fiber collector should be at least as high as the amount of sample delivered to the rotatable chamber. In an embodiment, the fiber collector has an outer diameter of 2 inches or less, 1.5 inches or less, 1 inch, or less 0.75 inches or less, or 0.5 inches or less.

In an embodiment, the fiber collector is removable from the rotatable chamber. For example, the fiber collector may be uncoupled from a cap that closes off the rotatable chamber and removed from the rotatable chamber. This allows for the fibers lodged in the fiber collector to be removed from the inner chamber prior to recovery the desired constituents of the sample that remain in the rotatable chamber after operation.

In an embodiment, the rotatable chamber further comprises a wiper. During operation of the device fibers may string together on the fiber collector and extend beyond the outer diameter of the fiber collector. These fibers which "hang" off of the fiber collector may trap desirable elements of the sample, such as non-fibrous fat tissue. The function of the wiper is to disrupt these hanging fibers to cause the desirable fat tissue to be released from the fibers. For example, as the fiber collector rotates some of the fibers that hang off of the fiber collector hit against the wiper, which disrupts the fibers and dislodges the non-fibrous tissue. The wiper may take any suitable form. In an embodiment, the wiper is a cylindrical rod.

In an embodiment, the wiper is present proximate the fiber collector in a position that the wiper will not contact the fiber collector during operation of the device. In an embodiment, the fiber collector is coaxial to the longitudinal axis and the wiper is present at a radius from the longitudinal axis that is greater than the outermost radius of the fiber collector.

In an embodiment the fiber collector rotates while the wiper remains stationary. In an embodiment, the wiper rotates while the fiber collector remains stationary. In an embodiment, the wiper rotates about the fiber collector at a different revolution frequency than the fiber collector.

The rotatable chamber further comprises a screen. The screen is configured to substantially retain a certain type of tissue on the interior of the screen while allowing liquids to pass through. In an embodiment, the screen is configured to retain fat tissue on its inner surface. In an embodiment the screen is cylindrical. In an embodiment, a surface of the screen is substantially parallel to the inner surface of the sidewall. In an embodiment, the screen is frustoconical. In an embodiment, the screen rotates along with the rotatable chamber. In an embodiment, the screen is stationary relative to the rotatable chamber. In an embodiment, the screen rotates at a revolution frequency that is less that the revolution frequency of the rotatable chamber.

In an embodiment, the screen projects away from the base. In an embodiment, the screen extends concentrically around the longitudinal axis of the rotatable chamber. In an embodiment, the screen is mesh-like. The screen may be a metal or polymer wire material, or a perforated sheet having openings of sufficiently size to allow for the passage of fluid while inhibiting desirable material to pass through.

The rotatable chamber comprises a first outlet. In an embodiment, the first outlet is located proximate the base of the rotatable chamber, such as in the sidewall near the base of the rotatable chamber, or in the base of the rotatable chamber proximate the sidewall. In an embodiment, the first outlet is located further from the longitudinal axis than the screen, such that material must pass through the screen to reach the first outlet.

In an embodiment, the first outlet is the opening of a first channel. The first channel serves as the passageway by which undesired liquids in the sample, such as blood, oil, or tumescent fluid, are dispensed from the rotatable chamber after being separated from other components of the sample. In an embodiment, the first channel is configured such that liquid present in the sample may leak out of the rotatable chamber through the first channel. The rate of liquid moving through the first channel may increase during rotation of the rotatable chamber.

In an embodiment, the first channel begins at the first outlet and ends at a collection container. In an embodiment, the first channel is in fluid communication with a collection container. The collection container collects fluids that are expelled by centrifugation of the sample. In an embodiment, the device comprises a collection container. In an embodiment, a collection container is contained within the device or within the outer enclosure, such as shown in Fig. 1. In an embodiment, the collection container is separately removable from the device.

In an embodiment, the first channel begins at the first outlet and ends outside of the outer enclosure. In an embodiment, the device does not comprise a collection container. In such embodiments, the first channel extends beyond the outer enclosure and may be directed into a container provided by the user.

In an embodiment, the first outlet is in fluid communication with a first valve that determines whether liquid may flow from the first outlet. In an embodiment, the valve is manually operable by the user. In an embodiment, the valve operates automatically as a result of the operation of the device.

In an embodiment, the valve comprises a dynamic seal. A dynamic seal is a seal that is normally in the closed position, thereby restricting flow through the channel, and opens in response to the force exerted on the dynamic seal. The force may be exerted on the dynamic seal by the sample or may be exerted on the dynamic seal by the rotation of the device. In an embodiment, the dynamic seal comprises an o-ring positioned in the channel.

In an embodiment, the channel is configured such that its path restricts the flow of liquid through the channel. In an embodiment, the channel comprises a path that at some point is pitched toward the end of the rotatable chamber that has a smaller diameter. In this way, components of the sample will generally remain in the rotatable chamber until the time at which the centrifugal force on the sample is substantial enough to direct components of the sample through the channel. In an embodiment, the channel comprises a serpentine path. In an embodiment, the channel is substantially straight and angled upward toward the end of the rotatable chamber having a smaller diameter.

In an embodiment, the rotatable chamber comprises a first outlet at a first radial distance from the longitudinal axis, a screen at a second radial distance from the longitudinal axis, a wiper at a third radial distance from the longitudinal axis, and a fiber collector having an outer edge at a fourth radial distance from the longitudinal axis, wherein the fourth radial distance is smaller than the third radial distance, the third radial distance is smaller than the second radial distance, and the second radial distance is smaller than the first radial distance.

In an embodiment, the rotatable chamber further comprises a second outlet at a different radial distance from the longitudinal axis than the first outlet. The second outlet may be the opening of a second channel. The second channel may comprise a second valve. Further outlets, channels, and valves may also be present.

In an embodiment, the device incorporates one or more vents (not shown) to allow for fluid displacement. One or more vents may provide for air to enter the chamber from which a fluid is displaced, or for air to leave a chamber from which fluid enters.

In an embodiment, the inner chamber comprises a trap. A trap is region at the bottom of the rotatable chamber that traps certain constituents of the sample during rotation of the rotatable chamber such that the trapped constituents are inhibited from remixing with other constituents of the sample after rotation is stopped. In an embodiment, the rotatable chamber comprises an oil trap for trapping oil near the base of the rotatable chamber.

In an embodiment, a filter is positioned at a distance above the base of the rotatable chamber, thereby forming a trap. In an embodiment, the filter is shaped like a disc. Such an embodiment is shown in Fig. 1. In an embodiment, the filter is made of metal, such as stainless steel. In an embodiment, the filter is made of a plastic, such as nylon. In an embodiment, the filter has an average opening size is of at least 0.025 mm or 0.05 mm. In an embodiment, the filter to has an average opening size of at most 0.25 mm, 0.2 mm, 0.15 mm, or 0.13 mm.

In an embodiment, the device comprises a drive unit, which serves as a rotation source for the rotatable chamber. The drive unit preferably couples to the processing unit. The drive unit comprises means to rotate the rotatable chamber. The drive unit preferably comprises an electric motor configured to rotate the rotatable chamber, but may also contains a hand crank or any other means to rotate the rotatable chamber that may be known to a person skilled in the art. In an embodiment, the drive unit comprises a hand crank and a spring and is configured so that the drive unit can be would up and the spring released to rotate the rotatable chamber. In an embodiment, the drive unit is separable from the processing unit, such that the drive unit may be reused without substantial cleaning or sterilization. In an embodiment, the processing unit is single-use or may be cleaned and sterilized, such that it may be reused.

In an embodiment, the drive unit is configured to rotate the rotatable chamber at multiple speeds. In an embodiment, the drive unit is configured to rotate the rotatable chamber such that the g-force acting on a sample within the rotatable chamber is 1000 g or less, 8000 g or less, or 5000 g or less. In an embodiment, the drive unit is configured to oscillate the rotatable chamber, such as a washing machine. In an embodiment, the drive unit is configured to oscillate in a sinusoidal wave pattern. In an embodiment, the drive unit is configured to oscillate in a square wave pattern. The drive unit may comprise manually operated controls, such as buttons, to allow an operator to direct the drive unit to rotate the rotatable chamber in a certain way, such as at a certain direction or certain speed.

In an embodiment, a process for processing a sample comprises the steps of:
a. introducing a sample into the rotatable chamber,
b. rotating the rotatable chamber, thereby stratifying the sample into constituents based on the density of the constituents,
c. removing a first fluid constituent from the sample via the first outlet in the rotatable chamber,
d. recovering a desired constituent of the sample from the rotatable chamber.

In an embodiment, the process further comprises the step of rotating a fiber collector. In an embodiment, the process further comprises the step of separating fibers from the constituent in the rotatable chamber by removing the fiber collector prior to recovering the desired constituent.

In an embodiment, the process further comprises the step of adding a cleaning solution after removing a first fluid constituent from the sample and prior to recovering the desired constituent of the sample. In an embodiment, the cleaning solution is a saline solution, a lactated ringers solution, or a phosphate buffered saline solution. In an embodiment, the device is configured to allow for addition of the cleaning solution while the rotatable chamber is rotating. In an embodiment, the process further comprises oscillating the rotatable chamber after adding the cleaning solution. In an embodiment, the process further comprises rotating the rotatable chamber, thereby stratifying the sample into constituents based on the density of the constituents, and removing a second fluid constituent form the sample via the first outlet in the rotatable chamber, wherein the second fluid constituent comprises the cleaning solution. In an embodiment, the process further comprises the step of removing the fibrous content of the sample by independently rotating the fiber collector to capture the fibrous content.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. While certain optional features are described as embodiments of the invention, the description is meant to encompass and specifically disclose all combinations of these embodiments unless specifically indicated otherwise or physically impossible.

### Additional Description of Certain Exemplary Embodiments

1. A device for processing adipose tissue comprising:
   a. a rotatable chamber having a longitudinal axis around which the rotatable chamber is arranged to be rotated, and a first end and a second end, with a side wall extending therebetween, wherein said first end has a diameter smaller than a diameter of said second end, the rotatable chamber comprising within:
      i. a fiber collector;
      ii. a first outlet at a first radial distance from said longitudinal axis;
      iii. a screen projecting from the second end and configured to restrict the passage of a constituent of adipose tissue therethrough, wherein the screen meets the second end at a second radial distance from the longitudinal axis, and wherein the second radial distance is less than the first radial distance;
   b. a drive unit configured to rotate the rotatable chamber about the longitudinal axis, thereby producing a centrifugal field, whereupon when a sample of adipose tissue is present in the rotatable chamber, the sample stratifies into at least two constituent layers as a function of the differing specific gravities of the constituents.
2. The device according to any one of the preceding exemplary embodiments, wherein the rotatable chamber is tubular.
3. The device according to any one of the preceding exemplary embodiments, wherein the rotatable chamber is tapered.
4. The device according to any one of the preceding exemplary embodiments, wherein the rotatable chamber is at least partially transparent.
5. The device according to any one of the preceding exemplary embodiments, wherein the device comprises a processing unit comprising the rotatable unit and a drive unit
6. The device according to any one of the preceding exemplary embodiments, wherein the rotatable chamber is enclosed by an outer enclosure.
7. The device according to any one of the preceding exemplary embodiments, wherein the processing unit comprises further comprises an outer enclosure.
8. The device according to any one of the preceding exemplary embodiments, wherein the outer enclosure is arranged to receive a biologic mixture from the rotatable chamber.
9. The device according to any one of the preceding exemplary embodiments, wherein the outer enclosure is arranged coaxially upon the central longitudinal axis of the rotatable chamber.
10. The device according to any one of the preceding exemplary embodiments, wherein the second end is the base of the rotatable chamber.
11. The device according to any one of the preceding exemplary embodiments, wherein the processing unit or rotatable chamber comprises a port to allow a sample to be placed into the rotatable chamber.
12. The device according to any one of the preceding exemplary embodiments, wherein the port is configured to receive the tip of a syringe.
13. The device according to any one of the preceding exemplary embodiments, wherein the port is configured to receive a catheter.
14. The device according to any one of the preceding exemplary embodiments, wherein the port comprises a cap that can be opened to allow input of the sample and closed to seal the processing unit.
15. The device according to any one of the preceding exemplary embodiments, wherein the fiber collector is configured to collect fibers from a sample.
16. The device according to any one of the preceding exemplary embodiments, wherein the fiber collector is rotatable.
17. The device according to any one of the preceding exemplary embodiments, wherein the fiber collector is rotatable about its central axis.
18. The device according to any one of the preceding exemplary embodiments, wherein the fiber collector is rotatable about the longitudinal axis of the rotatable chamber.
19. The device according to any one of the preceding exemplary embodiments, wherein fiber collector is connected to the same rotation source as the rotatable chamber.
20. The device according to any one of the preceding exemplary embodiments, wherein the fiber collector is connected to the same rotation source as the rotatable chamber and is configured to rotate slower than the rotation source of the rotatable chamber.
21. The device according to any one of the preceding exemplary embodiments, wherein fiber collector is connected to a different rotation source than the rotation source connected to the rotatable chamber.
22. The device according to any one of the preceding exemplary embodiments, wherein fiber collector is connected to its own rotation source.
23. The device according to any one of the preceding exemplary embodiments, wherein the rotation source coupled to the fiber collector is present at the top of the processing unit and may be decoupled from the fiber collector and reused.
24. The device according to any one of the preceding exemplary embodiments, wherein the fiber collector has the same revolution frequency as the rotatable chamber.
25. The device according to any one of the preceding exemplary embodiments, wherein the fiber collector is configured to rotate and at a slower revolution frequency than the rotatable chamber.
26. The device according to any one of the preceding exemplary embodiments, wherein the fiber collector is configured to rotate at a faster revolution frequency than the rotatable chamber.
27. The device according to any one of the preceding exemplary embodiments, wherein the fiber collector is present coaxially with or proximate to the longitudinal axis of the rotatable chamber.
28. The device according to any one of the preceding exemplary embodiments, wherein the fiber collector is present at a fifth radial distance from the longitudinal axis.
29. The device according to any one of the preceding exemplary embodiments, wherein the fiber collector is a brush.
30. The device according to any one of the preceding exemplary embodiments, wherein the fiber collector is a comb.
31. The device according to any one of the preceding exemplary embodiments, wherein the height of the fiber collector should be at least as high as the amount of sample delivered to the rotatable chamber.
32. The device according to any one of the preceding exemplary embodiments, wherein the fiber collector has an outer diameter of 2 inches or less, 1.5 inches or less, 1 inch, or less 0.75 inches or less, or 0.5 inches or less.
33. The device according to any one of the preceding exemplary embodiments, wherein the fiber collector is removable from the rotatable chamber.
34. The device according to any one of the preceding exemplary embodiments, wherein the fiber collector may be uncoupled from a cap that closes off the rotatable chamber and removed from the rotatable chamber.
35. The device according to any one of the preceding exemplary embodiments, wherein the rotatable chamber further comprises a wiper.
36. The device according to any one of the preceding exemplary embodiments, wherein the wiper disrupts fibers hanging off of the fiber collector to cause the desirable fat tissue to be released from the fibers.
37. The device according to any one of the preceding exemplary embodiments, wherein the wiper is a cylindrical rod.
38. The device according to any one of the preceding exemplary embodiments, wherein the wiper is present proximate the fiber collector in a position that the wiper will not contact the fiber collector during operation of the device.
39. The device according to any one of the preceding exemplary embodiments, wherein the fiber collector is coaxial to the longitudinal axis and the wiper is present at a radius from the longitudinal axis that is greater than the outermost radius of the fiber collector.
40. The device according to any one of the preceding exemplary embodiments, wherein the fiber collector rotates while the wiper remains stationary.
41. The device according to any one of the preceding exemplary embodiments, wherein the wiper rotates while the fiber collector remains stationary.
42. The device according to any one of the preceding exemplary embodiments, wherein the wiper rotates about the fiber collector at a different revolution frequency than the fiber collector.
43. The device according to any one of the preceding exemplary embodiments, wherein the screen is configured to substantially retain a certain type of tissue on the interior of the screen while allowing liquids to pass through.
44. The device according to any one of the preceding exemplary embodiments, wherein the screen is configured to retain fat tissue on its inner surface.
45. The device according to any one of the preceding exemplary embodiments, wherein the screen is cylindrical.
46. The device according to any one of the preceding exemplary embodiments, wherein a surface of the screen is substantially parallel to the inner surface of the sidewall.
47. The device according to any one of the preceding exemplary embodiments, wherein the screen is frustoconical.
48. The device according to any one of the preceding exemplary embodiments, wherein the screen rotates along with the rotatable chamber.
49. The device according to any one of the preceding exemplary embodiments, wherein the screen is stationary relative to the rotatable chamber.
50. The device according to any one of the preceding exemplary embodiments, wherein the screen rotates at a revolution frequency that is less that the revolution frequency of the rotatable chamber.
51. The device according to any one of the preceding exemplary embodiments, wherein the screen projects away from the base.
52. The device according to any one of the preceding exemplary embodiments, wherein the screen extends concentrically around the longitudinal axis of the rotatable chamber.
53. The device according to any one of the preceding exemplary embodiments, wherein the screen is mesh-like.
54. The device according to any one of the preceding exemplary embodiments, wherein the screen is made of metal or polymer wire material, or a perforated sheet having openings of sufficiently size to allow for the passage of fluid while inhibiting desirable material to pass through.
55. The device according to any one of the preceding exemplary embodiments, wherein the first outlet is located proximate the base of the rotatable chamber.
56. The device according to any one of the preceding exemplary embodiments, wherein the first outlet is located in the sidewall near the base of the rotatable chamber.
57. The device according to any one of the preceding exemplary embodiments, wherein the first outlet is located in the base of the rotatable chamber proximate the sidewall.
58. The device according to any one of the preceding exemplary embodiments, wherein the first outlet is located further from the longitudinal axis than the screen, such that material must pass through the screen to reach the first outlet.
59. The device according to any one of the preceding exemplary embodiments, wherein the first outlet is the opening of a first channel.
60. The device according to any one of the preceding exemplary embodiments, wherein the first channel serves as the passageway by which undesired liquids in the sample are dispensed from the rotatable chamber after being separated from other components of the sample.
61. The device according to any one of the preceding exemplary embodiments, wherein the first channel is configured such that liquid present in the sample may leak out of the rotatable chamber through the first channel.
62. The device according to any one of the preceding exemplary embodiments, wherein the rate of liquid moving through the first channel may increase during rotation of the rotatable chamber.
63. The device according to any one of the preceding exemplary embodiments, wherein the first channel begins at the first outlet and ends at a collection container.
64. The device according to any one of the preceding exemplary embodiments, wherein the first channel is in fluid communication with a collection container.
65. The device according to any one of the preceding exemplary embodiments, wherein the device comprises a collection container.
66. The device according to any one of the preceding exemplary embodiments, wherein the collection container is contained within the device.
67. The device according to any one of the preceding exemplary embodiments, wherein the collection container is contained within the outer enclosure.
68. The device according to any one of the preceding exemplary embodiments, wherein the collection container is separately removable from the device.
69. The device according to any one of the preceding exemplary embodiments, wherein the first channel begins at the first outlet and ends outside of the outer enclosure.
70. The device according to any one of the preceding exemplary embodiments, wherein the device does not comprise a collection container.
71. The device according to any one of the preceding exemplary embodiments, wherein the first channel extends beyond the outer enclosure and may be directed into a container provided by the user.
72. The device according to any one of the preceding exemplary embodiments, further comprising a first valve.
73. The device according to any one of the preceding exemplary embodiments, wherein the first outlet is in fluid communication with a first valve that determines whether liquid may flow from the first outlet or through the first channel.
74. The device according to any one of the preceding exemplary embodiments, wherein the valve is manually operable by the user.
75. The device according to any one of the preceding exemplary embodiments, wherein the valve operates automatically as a result of the operation of the device.
76. The device according to any one of the preceding exemplary embodiments, wherein the valve comprises a dynamic seal.
77. The device according to any one of the preceding exemplary embodiments, wherein the dynamic seal is normally in the closed position, thereby restricting flow through the channel, and opens in response to the force exerted on the dynamic seal.
78. The device according to any one of the preceding exemplary embodiments, wherein the dynamic seal comprises an o-ring positioned in the channel.
79. The device according to any one of the preceding exemplary embodiments, wherein the channel is configured such that its path restricts the flow of liquid through the channel.
80. The device according to any one of the preceding exemplary embodiments, wherein the channel comprises a path that at some point is pitched toward the end of the rotatable chamber that has a smaller diameter.
81. The device according to any one of the preceding exemplary embodiments, wherein the channel comprises a serpentine path.
82. The device according to any one of the preceding exemplary embodiments, wherein the channel is substantially straight and angled upward toward the end of the rotatable chamber having a smaller diameter.
83. The device according to any one of the preceding exemplary embodiments, wherein the rotatable chamber comprises a first outlet at a first radial distance from the longitudinal axis, a screen at a second radial distance from the longitudinal axis, a wiper at a third radial distance from the longitudinal axis, and a fiber collector having an outer edge at a fourth radial distance from the longitudinal axis, wherein the fourth radial distance is smaller than the third radial distance, the third radial distance is smaller than the second radial distance, and the second radial distance is smaller than the first radial distance.
84. The device according to any one of the preceding exemplary embodiments, wherein the rotatable chamber further comprises a second outlet at a different radial distance from the longitudinal axis than the first outlet.
85. The device according to any one of the preceding exemplary embodiments, wherein the rotatable chamber further comprises a second channel with its opening at the second outlet.
86. The device according to any one of the preceding exemplary embodiments, wherein the second channel comprises a second valve.
87. The device according to any one of the preceding exemplary embodiments, wherein the device comprises one or more vents to allow for fluid displacement in the rotatable chamber.
88. The device according to any one of the preceding exemplary embodiments, wherein the inner chamber comprises a trap.
89. The device according to any one of the preceding exemplary embodiments, wherein the rotatable chamber comprises an oil trap for trapping oil near the base of the rotatable chamber.
90. The device according to any one of the preceding exemplary embodiments, wherein a filter is positioned at a distance above the base of the rotatable chamber, thereby forming a trap.
91. The device according to any one of the preceding exemplary embodiments, wherein the filter is shaped like a disc.
92. The device according to any one of the preceding exemplary embodiments, wherein the filter has an average opening size is of at least 0.025 mm or 0.05 mm.
93. The device according to any one of the preceding exemplary embodiments, wherein the filter to has an average opening size of at most 0.25 mm, 0.2 mm, 0.15 mm, or 0.13 mm.
94. The device according to any one of the preceding exemplary embodiments, wherein the drive unit couples to the processing unit.
95. The device according to any one of the preceding exemplary embodiments, wherein the drive unit comprises an electric motor configured to rotate the rotatable chamber
96. The device according to any one of the preceding exemplary embodiments, wherein the drive unit comprises a hand crank to rotate the rotatable chamber.
97. The device according to any one of the preceding exemplary embodiments, wherein the drive unit comprises a hand crank and a spring and is configured so that the drive unit can be would up and the spring released to rotate the rotatable chamber.
98. The device according to any one of the preceding exemplary embodiments, wherein the drive unit is separable from the processing unit such that the drive unit may be reused without substantial cleaning or sterilization.
99. The device according to any one of the preceding exemplary embodiments, wherein the processing unit is single-use or may be cleaned and sterilized, such that it may be reused.
100. The device according to any one of the preceding exemplary embodiments, wherein the drive unit is configured to rotate the rotatable chamber at multiple speeds.
101. The device according to any one of the preceding exemplary embodiments, wherein the drive unit is configured to rotate the rotatable chamber such that the g-force acting on a sample within the rotatable chamber is 1000 g or less, 8000 g or less, or 5000 g or less.
102. The device according to any one of the preceding exemplary embodiments, wherein the drive unit is configured to oscillate the rotatable chamber.
103. The device according to any one of the preceding exemplary embodiments, wherein the drive unit is configured to oscillate in a sinusoidal wave pattern.
104. The device according to any one of the preceding exemplary embodiments, wherein the drive unit is configured to oscillate in a square wave pattern.
105. The device according to any one of the previous exemplary embodiments, wherein the device is configured to allow for addition of a cleaning solution while the rotatable chamber is rotating.
106. A process for processing a sample comprising the steps of:
   a. providing a device according to any one of the preceding exemplary embodiments,
   b. introducing a sample into the rotatable chamber,
   c. rotating the rotatable chamber, thereby stratifying the sample into constituents based on the density of the constituents,
   d. removing a first fluid constituent from the sample via the first outlet in the rotatable chamber, and
   e. recovering a desired constituent of the sample from the rotatable chamber.
107. The process according to the previous exemplary embodiment, further comprising the step of rotating the fiber collector.
108. The process according to any one of the previous exemplary embodiments, further comprising the step of separating fibers from the constituent in the rotatable chamber by removing the fiber collector prior to recovering the desired constituent.
109. The process according to any one of the previous exemplary embodiments, further comprising the step of adding a cleaning solution after removing a first fluid constituent from the sample and prior to recovering the desired constituent of the sample.
110. The process according to any one of the previous exemplary embodiments, wherein the cleaning solution is a saline solution, a lactated ringers solution, or a phosphate buffered saline solution.
111. The process according to any one of the previous exemplary embodiments, further comprising the step of adding a cleaning solution to the rotatable chamber while the rotatable chamber is rotating.
112. The process according to any one of the previous exemplary embodiments, further comprising the step of oscillating the rotatable chamber after adding the cleaning solution.
113. The process according to any one of the previous exemplary embodiments, further comprising the step of rotating the rotatable chamber, thereby stratifying the sample into constituents based on the density of the constituents, and removing a second fluid constituent form the sample via the first outlet in the rotatable chamber, wherein the second fluid constituent comprises the cleaning solution.
114. The process according to any one of the previous exemplary embodiments, wherein the process further comprises the step of removing the fibrous content of the sample by independently rotating the fiber collector to capture the fibrous content.

## Claims

1. A device for processing adipose tissue comprising:
a. a rotatable chamber having a longitudinal axis around which the rotatable chamber is arranged to be rotated, and a first end and a second end, with a side wall extending therebetween, wherein said first end has a diameter smaller than a diameter of said second end, the rotatable chamber comprising within:
i. a fiber collector;
ii. a first outlet at a first radial distance from said longitudinal axis;
iii. a screen projecting from the second end and configured to restrict the passage of a constituent of adipose tissue therethrough, wherein the screen meets the second end at a second radial distance from the longitudinal axis, and wherein the second radial distance is less than the first radial distance;
b. a drive unit configured to rotate the rotatable chamber about the longitudinal axis, thereby producing a centrifugal field, whereupon when a sample of adipose tissue is present in the rotatable chamber, the sample stratifies into at least two constituent layers as a function of the differing specific gravities of the constituents.

2. The device according to any one of the preceding claims, wherein the fiber collector is rotatable.

3. The device according to any one of the preceding claims, wherein the fiber collector is a brush or a comb.

4. The device according to any one of the preceding claims, wherein the rotatable chamber further comprises a wiper configured to disrupt fibers hanging off of the fiber collector to cause the desirable fat tissue to be released from the fibers, and wherein the fiber collector rotates while the wiper remains stationary.

5. The device according to any one of the preceding claims, wherein the rotatable chamber further comprises a wiper configured to disrupt fibers hanging off of the fiber collector to cause the desirable fat tissue to be released from the fibers, wherein the fiber collector is coaxial to the longitudinal axis and the wiper is present at a radius from the longitudinal axis that is greater than the outermost radius of the fiber collector, and wherein the fiber collector rotates about the longitudinal axis of the rotatable chamber while the wiper remains stationary.

6. The device according to any one of the preceding claims, wherein the screen is configured to retain fat tissue on its inner surface.

7. The device according to any one of the preceding claims, wherein the first outlet is located further from the longitudinal axis than the screen, such that material must pass through the screen to reach the first outlet.

8. The device according to any one of the preceding claims, wherein the first outlet is the opening of a first channel that is in fluid communication with a collection container.

9. The device according to any one of the preceding claims, wherein the first outlet is in fluid communication with a first valve that determines whether liquid may flow from the first outlet or through the first channel.

10. The device according to claim 9, wherein the valve operates automatically as a result of the operation of the device.

11. The device according to claim 9 or 10, wherein the valve comprises a dynamic seal, wherein the dynamic seal is normally in the closed position, thereby restricting flow through the channel, and opens in response to the force exerted on the dynamic seal.

12. The device according to any one claims 4-11, wherein the rotatable chamber comprises the first outlet at a first radial distance from the longitudinal axis, the screen at a second radial distance from the longitudinal axis, the wiper at a third radial distance from the longitudinal axis, and the fiber collector having an outer edge at a fourth radial distance from the longitudinal axis, wherein the fourth radial distance is smaller than the third radial distance, the third radial distance is smaller than the second radial distance, and the second radial distance is smaller than the first radial distance.

13. The device according to any one of the preceding claims, wherein the rotatable chamber comprises an oil trap for trapping oil near the base of the rotatable chamber, wherein a filter is positioned at a distance above the base of the rotatable chamber, thereby forming the oil trap.

14. The device according to any one of the preceding claims, wherein the drive unit is configured to oscillate the rotatable chamber.

15. A process for processing a sample comprising the steps of:
a. providing a device according to any one of the preceding claims,
b. introducing a sample into the rotatable chamber,
c. rotating the rotatable chamber and the fiber collector, thereby stratifying the sample into constituents based on the density of the constituents,
d. removing a first fluid constituent from the sample via the first outlet in the rotatable chamber, and
e. separating fibers from the constituent in the rotatable chamber by removing the fiber collector prior to recovering a desired constituent,
f. recovering the desired constituent of the sample from the rotatable chamber.
